(19) Europäisches Patentamt
European Patent Office
Office européen des brevets

(11) **EP 1 894 532 B1**

(12) **EUROPEAN PATENT SPECIFICATION**

(45) Date of publication and mention
of the grant of the patent:
**03.03.2010 Bulletin 2010/09**

(51) Int Cl.:
*A61B 17/22* *(2006.01)* *A61B 17/32* *(2006.01)*

(21) Application number: **07013285.7**

(22) Date of filing: **06.07.2007**

(54) **Surgical instruments**

Chirurgische Instrumente

Instruments chirurgicaux

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.08.2006 US 468399**

(43) Date of publication of application:
**05.03.2008 Bulletin 2008/10**

(73) Proprietor: **OLYMPUS MEDICAL SYSTEMS CORP.**
**Shibuya-ku**
**Tokyo 151-0072 (JP)**

(72) Inventor: **Kimura, Kenichi**
**Hachioji-shi**
**Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**EP-A- 1 810 630** **WO-A-02/056805**
**JP-A- 3 111 037** **JP-A- 2002 306 507**
**US-A1- 2003 040 672**

## Description

This invention relates to a surgical instrument to be used for performing a treatment of incision and coagulation on living body tissue by means of a high frequency current and ultra-sonic vibration.

Treating instruments that are being popularly used include electric knives utilizing a high frequency current that can stanch (coagulate) blood in and at the same time incise living body tissue as well as operating knives. Japanese Patent Application Publication No. 2002-306507 discloses a surgical instrument comprising a knife tip electrode that has an electrode section for treating living body tissue by utilizing a high frequency current, a high frequency current supply section for supplying a high frequency current to the knife tip electrode and an ultrasonic oscillation supply section for supplying ultrasonic oscillations to the knife tip electrode.

The above-cited surgical instrument is devised to make the knife tip electrode ultrasonically oscillate as long as a high frequency current is supplied to the knife tip electrode in order to prevent living body tissue from being scorched by the knife tip electrode. When using this surgical instrument, an operator is required to set the instrument ready for use in advance by way of cumbersome operations depending on the type of living body tissue (muscles, fat tissues, parenchymal organs) and conditions (a condition in which the amount of body fluids is high, a very dry condition, and the like) in order to realize predetermined optimal high frequency and ultrasonic outputs.

Document US 2003/0040672 A1 concerns a surgical operation apparatus comprising a tip member for outputting ultrasound vibration and/or high-frequency coagulation current, a hand piece provided with a tip member, a circuit for supplying the high-frequency coagulation current to the tip member and a circuit for supplying the ultrasound vibration to the tip member.

Document WO 02/056805 A2 concerns a surgical instrument outputting ultrasonic waves and high frequency waves. In one embodiment, impedance monitoring is provided for keeping impedance levels within acceptable limits by controlling the power supplied by a RF generator.

Therefore, it is an object of the present invention to provide a surgical instrument that can be operated with ease and is adapted to give an optimal treatment regardless of the type and condition of living body tissue and a surgical instrument driving method to be used for such an instrument.

According to the present invention, the above object is achieved by providing a surgical instrument having the features of claim 1.

The surgical instrument includes: a hand piece equipped with a treatment section arranged at the tip side thereof to treat living body tissue and including a probe to be supplied with a high frequency current according to a directive and an ultrasonic transducer rigidly secured to the probe and adapted to ultrasonically activate the probe; a high frequency drive circuit for supplying a high frequency current to the probe; and an ultrasonic transducer drive circuit for driving the ultrasonic transducer, the ultrasonic transducer drive circuit being adapted to control the amplitude of ultrasonic vibration at the treatment section according to the magnitude of the impedance value as detected by the high frequency drive circuit at the time of outputting a high frequency current.

There is also provided a method of driving a surgical instrument for supplying a high frequency current and/or ultrasonic vibration to the treatment section of a probe for treating living body tissue and controlling the amplitude of the ultrasonic vibration being supplied to the treatment section according to the magnitude of the impedance value between the living body tissue and the treatment section as detected by the high frequency drive circuit for supplying a high frequency current to the probe at the time of outputting the high frequency current.

The invention can be more fully understood from the following detailed description when taken in conjunction with the accompanying drawings, in which:

FIG. 1 is a schematic perspective view of a first embodiment of surgical instrument, showing the appearance thereof;

FIG. 2 is a schematic cross sectional view of the hand piece of the surgical instrument of FIG. 1;

FIG. 3 is a schematic illustration of a hook type treatment section;

FIG. 4 is a schematic illustration of a ball type treatment section;

FIG. 5 is a schematic block diagram of an ultrasonic drive unit and a high frequency drive unit;

FIG. 6 is a list of output modes of the surgical instrument;

FIG. 7 is a graph illustrating the impedance when the high frequency output exists and the output characteristic of the high frequency output when the highest output power level is selected;

FIG. 8 is a graph illustrating the impedance when the high frequency output exists and the output characteristic of the high frequency output when an output power level of 50W is selected;

FIG. 9 is a graph illustrating an impedance when a high frequency output exists and output characteristics in terms of magnitude of ultrasonic output as a function of impedance;

FIG. 10 is a graph illustrating the impedance when the high frequency output exists and an example of output characteristics in terms of the magnitude of the ultrasonic output as a function of the impedance;

FIG. 11 is a graph illustrating the impedance when the high frequency output exists and an example of output characteristics in terms of the magnitude of the ultrasonic output as a function of the impedance;

FIG. 12A is a schematic illustration of an image that can be displayed on one of the operation/display panels for a setting of operation;

FIG. 12B is a schematic illustration of another image that can be displayed on the other operation/display panel for the setting of FIG. 12A;

FIG. 13A is a schematic illustration of an image that can be displayed on one of the operation/display panels for another setting of operation;

FIG. 13B is a schematic illustration of another image that can be displayed on the other operation/display panel for the setting of FIG. 13A;

FIG. 14 is a graph illustrating the relationship between the high frequency output and electric impedance and between the ultrasonic output and the electric impedance;

FIG. 15 is a schematic perspective view of a second embodiment of surgical instrument, showing the appearance thereof;

FIG. 16A is a schematic front view of the treatment section of a third embodiment of surgical instrument;

FIG. 16B is a schematic plan view of the treatment section of the third embodiment of surgical instrument;

FIG. 17 is a schematic cross sectional view of the treatment section taken along line A-A in FIG. 16B; and

FIG. 18 is a schematic longitudinal cross sectional view of the treatment section of the third embodiment.

Now, embodiments of the present invention will be described in greater detail by referring to the accompanying drawings.

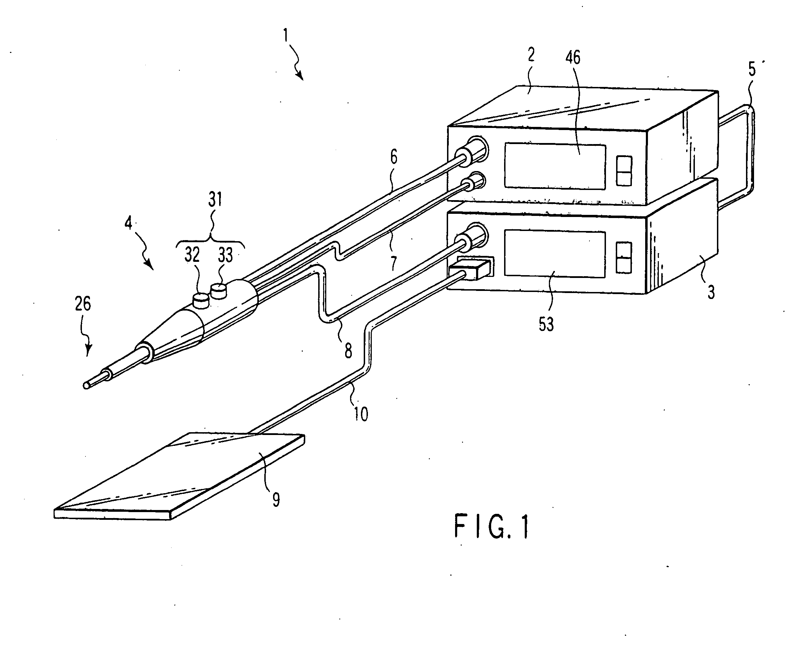

FIG. 1 is a schematic perspective view of a first embodiment of surgical instrument, showing the appearance thereof.

Referring to FIG. 1, the surgical instrument 1 comprises an ultrasonic drive unit 2, a high frequency drive unit 3 and a hand piece 4 as principal components thereof. The ultrasonic drive unit 2 and the high frequency drive unit 3 are connected to each other by way of a communication cable 5 and the hand piece 4 is connected to the ultrasonic drive unit 2 by way of an output connection cable 6 and an SW connection cable 7 and also to the high frequency drive unit 3 by means of a connector. Additionally, the hand piece 4 is connected to the high frequency drive unit 3 by way of an output connection cable 8. A bipolar plate 9 is connected to the high frequency drive unit 3 by way of a connection cable 10 and by means of a connector. The ultrasonic drive unit 2 and the high frequency drive unit 3 are provided with operation/display panels 46 and 53, respectively.

Now, the hand piece 4 will be described.

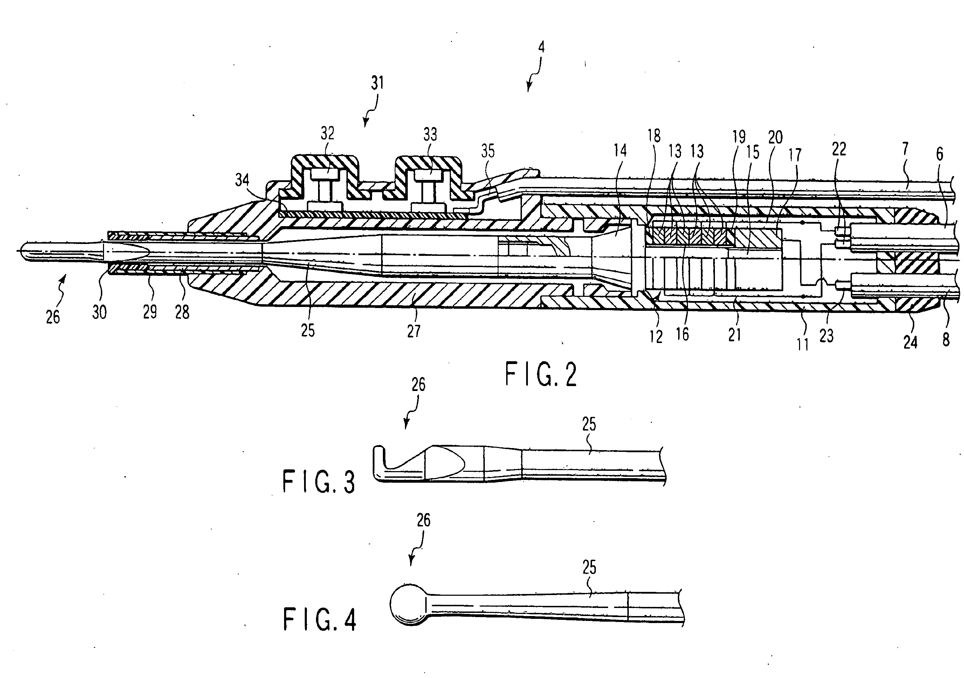

FIG. 2 is a schematic cross sectional view of the hand piece 4. Referring to FIG. 2, the hand piece 4 is provided at the tip side (at the side of probe 25) thereof with a sheath main body 27 and at the rear side thereof with a substantially cylindrical case 11. The case 11 and the sheath main body 27 are fit into each other. The case 11 and the sheath main body 27 are made of an insulating resin material having insulation properties. However, they may be made of some other material so long as it is insulating.

The output connection cables 6 and 8 are rigidly secured to the rear end of the case 11. More specifically, they are made to run through a protective elastic member 24 arranged integrally with the case 11 at the rear end of the latter and connected respectively to the ultrasonic drive unit 2 and the high frequency drive unit 3, which are described above by way of respective connectors.

An ultrasonic transducer (e.g., bolt-clamped Langevin type transducer) 12 is rigidly fitted to the case 11 to generate ultrasonic oscillations(vibration). The ultrasonic transducer 12 is provided with a plurality of ring-shaped piezoelectric elements 13 for transforming the electric power supplied from the ultrasonic drive unit 2 into ultrasonic oscillations. While a total of six piezoelectric elements 13 are arranged in a row in this embodiment, the number of piezoelectric elements may be appropriately determined according to the design specifications.

A horn 14 is arranged at the front end side of the row of the piezoelectric elements 13 to amplify the ultrasonic oscillations generated by the piezoelectric elements 13. The horn 14 is made of a metal material such as titanium, duralumin or stainless steel.

A bolt 15 is formed at the rear end side of the horn 14. The bolt 15 is driven into the rings of the piezoelectric elements 13 with a pipe-shaped insulating member 16 interposed between them. The piezoelectric elements 13 are rigidly secured in position as a metal-made nut 17 arranged at the rear end side of the row of the piezoelectric elements 13 and the bolt 15 are engaged with each other.

A ring-shaped insulating member 18 is interposed between the front end piezoelectric element 13 of the row and the horn 14. Similarly, a ring-shaped insulating member 19 is interposed between the rear end piezoelectric element 13 of the row and the nut 17. The piezoelectric elements 13 are electrically insulated relative to the horn 14 and the nut 17 by these insulating members 18, 19 and 16. The piezoelectric elements 13 are connected to a conductor 22 in the output connection cable 6 by way of metal-made bridges 20, 21. The piezoelectric elements 13 are supplied with ultrasonic drive power by way of the conductor 22. The nut 17 is connected to a conductor 23 in the output connection cable 8. The nut 17 and the horn 14 are supplied with a high frequency current by way of the conductor 23.

A probe 25 is screwed into the front end of the horn 14 as concluding member in order to transmit the ultrasonic oscillations amplified by the horn 14. Like the horn 14, the probe 25 is made of a metal material such as titanium, duralumin or stainless steel. A treatment section 26 is arranged at the front end of the probe 25 in order to treat living body tissue.

FIG. 2 shows a substantially flat spatula type treatment section 26 as an example. However, the spatula type treatment section 26 may be replaced by a hook type treatment section as shown in FIG. 3 or a ball type treatment section as shown in FIG. 4, which are not flat.

A metal pipe 28 and an insulating tube 29 that covers the metal pipe 28 are fitted to the front end of the sheath main body 27. A support member 30 is arranged at the front end of the metal pipe 28 to prevent the probe 25 and the metal pipe 28 from contacting with each other and becoming rickety relative to each other. The insulating member 29 and the support member 30 are made of a highly electrically insulating resin material such as PTFE.

The sheath main body 27 is provided with a first switch 32 and a second switch 33 that operate as hand switches 31. The first switch 32 and the second switch 33 are mounted on an electric circuit board 34 and contained in the sheath main body 27. A conductor 35 in the SW connection cable 7 is connected to a connection terminal (pad) of the electric circuit board 34 typically by soldering. The SW connection cable 7 extends externally from the rear end of the sheath main body 27 and connected to the ultrasonic drive unit 2 by means of a connector. With this arrangement, the electric signals of the first switch 32 and the second switch 33 (open, short-circuiting) are transmitted to the ultrasonic drive unit 2 by way of the SW connection cable 7.

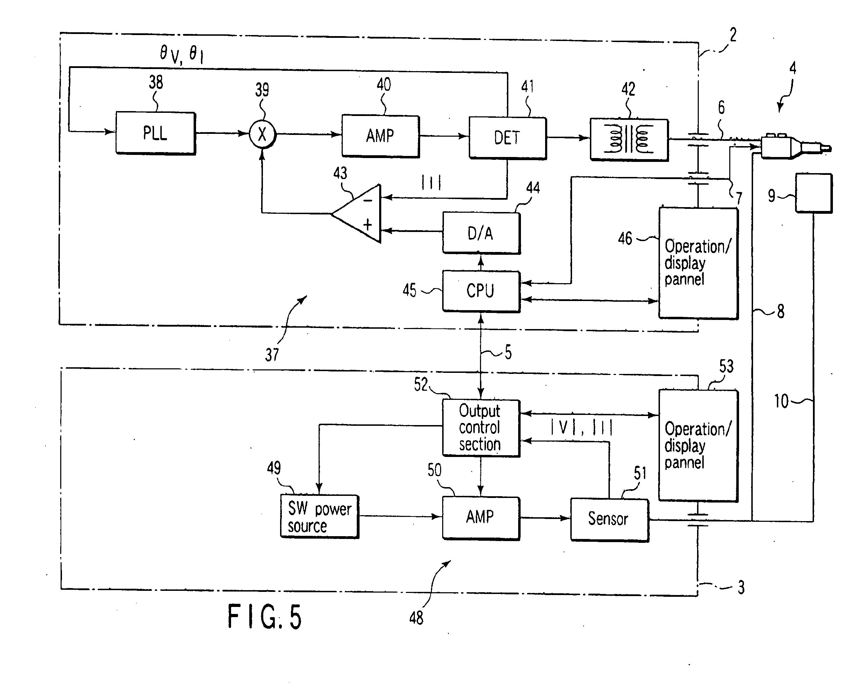

FIG. 5 is a schematic block diagram of the ultrasonic drive unit 2 and the high frequency drive unit 3.

Referring to FIG. 5, an ultrasonic transducer drive circuit 37 is contained in the ultrasonic drive unit 2. In the illustrated instance, a PLL control system and a current control system are employed respectively as resonance tracking system and amplitude control system.

The ultrasonic transducer drive circuit 37 includes a phase tracking circuit (PLL) 38 and an output circuit formed by connecting a voltage control amp (VCA) 39 which is a multiplier, a power amplifier (AMP) 40 for generating an electric current in order to energize the ultrasonic transducer 12, a voltage/current detecting section (DET) 41 and an output transformer 42 in series.

The hand piece 4 is connected to the output port of the output transformer 42 by way of the output connection cable 6 to which the hand piece 4 is connected by means of a connector. The ultrasonic transducer drive circuit 37 and the hand piece 4 are separated from each other by the output transformer 42 so that no DC component flow there. The phase tracking circuit (PLL) 38 is a circuit for tracking the resonance frequency of the ultrasonic transducer 12 and driving it at the resonance point.

The voltage/current detecting section (DET) 41 is connected to the phase tracking circuit (PLL) 38. Additionally, the voltage/current detecting section (DET) 41 includes a circuit for detecting the phase signal of the voltage and the current to be subjected to PLL or the magnitude of the current flowing to the ultrasonic transducer 12. The ultrasonic transducer drive circuit 37 includes a differential amplifier 43, a D/A converter 44 and a CPU 45. With this arrangement, the D/A converter 44 and the operation/display panel 46 are connected to the CPU 45.

The CPU 45 is connected to the first and second switches 32, 33 by way of the SW connection cable 7. The voltage/current detecting section (DET) 41 is connected to one of the input terminal of the differential amplifier 43, while the D/A converter 44 is connected to the other input terminal of the differential amplifier 43. The D/A converter 44 is adapted to output a signal for specifying an electric current that corresponds to the ultrasonic output conditions specified by the CPU 45.

Additionally, the output terminal of the differential amplifier 43 is connected to the voltage control amp (VCA) 39. The differential amplifier 43 compares the signal output from the D/A converter 44 and the magnitude of the electric current detected by the voltage/current detecting section (DET) 41 and operates to eliminate the difference, if any, before it supplies the amplified output to the voltage control amp (VCA) 39. With this arrangement, the magnitude of the voltage applied to the ultrasonic transducer 12 is controlled so as to make the electric current flowing to the ultrasonic transducer 12 satisfy the output conditions specified by the CPU 45.

A high frequency drive circuit 48 is contained in the high frequency drive unit 3. The high frequency drive circuit 48 is formed by connecting a variable voltage source (SW power source) 49 for supplying high frequency output power and control power, a power amplifier (AMP) 50 and a sensor 51 in series so as to operate as output circuit. The sensor 51 is connected to the hand piece 4 by way of the output connection cable 8 that is connected to the hand piece 4 by means of a connector and also to the bipolar plate 9 by way of the connection cable 10.

Additionally, an output control section 52 is arranged in the high frequency drive circuit 48. The sensor 51, the variable voltage source (SW power source) 49, the power amplifier (AMP) 50 and the operation/display panel 53 are connected to the output control section 52 so as to process the sensor signal and control the components.

The variable voltage source (SW power source) 49 generates high frequency output power and control power. The power amplifier (AMP) 50 amplifies high frequency power and rectifies the output waveform. The sensor 51 monitors the change in the high frequency output (voltage value, current value) and transmits monitor signals to the output control section 52. The output control section 52 transmits control signals to the variable voltage source (SW power source) 49 and the power amplifier (AMP) 50 according to the monitor signals from the sensor 51 so as to control the high frequency output. Note that the CPU 45 of the ultrasonic transducer drive circuit 37 and the output control section 52 of the high frequency drive circuit 48 are connected to each other by way of a communication cable 5 that can bidirectionally transmit signals. The CPU 45 transmits ON/OFF signals of the hand switch 31 to the output control section 52.

The output control section 52 transmits a signal indicating the magnitude of the impedance value (impedance value between the hand piece 4 and the bipolar plate 9) for high frequency output each time when it is computationally determined on an ad hoc basis or at arbitrarily predetermined time intervals according to the monitor signals (voltage value, current value) of the sensor 51.

The operation/display panel 46 of the ultrasonic drive unit 2 and the operation/display panel 53 of the high frequency drive unit 3 are connected to each other by way of the CPU 45, the communication cable 5 and the output control section 52. It is so arranged that the same selected items and same information are displayed on the operation/display panel 46 and the operation/display panel 53. Alternatively however, it may be so arranged that only necessary information are shared by the two operation/display panels and otherwise the two operation/display panels display different pieces of information.

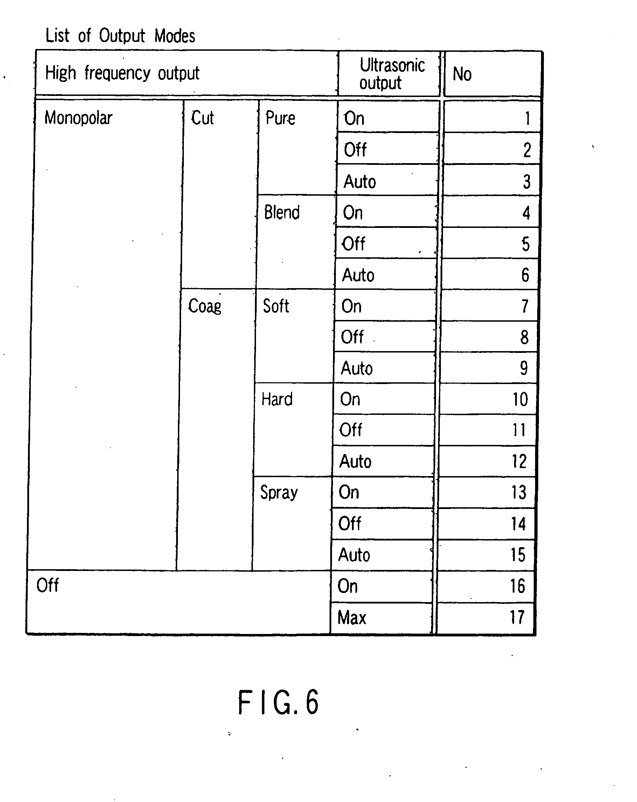

FIG. 6 is a list of output modes of the surgical instrument 1, shown as an example.

Like known monopolar type electric surgical knife apparatus, the high frequency drive unit 3 is adapted to provide a high frequency output in any of different output modes including a "Cut-Pure" mode (an incision output involving practically no hemostatic component (or requiring no coagulatory treatment)), a "Cut-Blend" mode (an incision output involving a hemostatic component to a certain extent), a "Coag-Soft" mode (a coagulation output involving a weak exfoliative component), a "Coag-Hard" mode (a coagulation output involving a strong exfoliative component) and a "Coag-Spray" mode (a coagulation output to be used for uniformly discharge-stanching blood over a large area of tissues). On the other had, the ultrasonic drive unit 2 is adapted to provide ultrasonic waves in any of different output modes including "On" mode (an ultrasonic output where the amplitude of ultrasonic oscillations is maintained at a selected level) and an "Auto" mode (an ultrasonic output where the amplitude of ultrasonic oscillations changes as a function of the impedance value at the time of high frequency output). Numbers are assigned to the combinations of the modes of high frequency outputs and those of ultrasonic outputs. The selected output modes are displayed when it is selected on the operation/display panel as will be described in greater detail hereinafter.

The surgical instrument 1 can combine any of the output modes for the high frequency output and any of the output modes for the ultrasonic output. Additionally, it is also possible to combine an "off" mode for the high frequency output (no output) and an "on" mode (an ultrasonic output where the amplitude of ultrasonic oscillations is maintained at a selected level) for the ultrasonic output or a "max" mode (an ultrasonic output where the amplitude of ultrasonic oscillations maintained at the largest level). In other words, it is possible to provide only an ultrasonic output.

It is also possible to combine any of the output mode for the high frequency output (e.g. a "Cut Pure" mode or a "Coag-Hard" mode) and an "Off" (no output) mode for the ultrasonic output. In other words, it is possible to provide only a high frequency output.

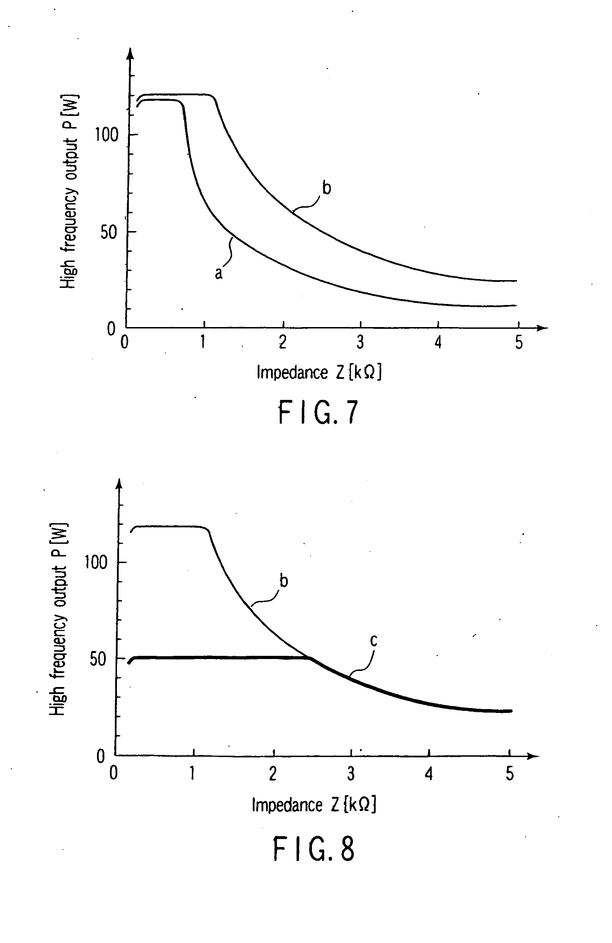

FIGS. 7 and 8 illustrate the output characteristic (load characteristic) of the high frequency output of this embodiment as examples.

In FIG. 7, the horizontal axis indicates the impedance at the time of high frequency output (the impedance between the hand piece 4 and the bipolar plate 9) and the vertical axis indicates the high frequency output at the time of selecting the largest output. In FIG. 7, characteristic curve a shows the output characteristic in a "Coag-Soft" mode while characteristic curve b shows the output characteristic in a "Coag-Hard" mode.

The high frequency output of the embodiment shows output characteristic that the high frequency output falls as the impedance rises in both a "Coag-Soft" mode and a "Coag-Hard" mode as in the case of known monopolar type electric knife apparatus. Note, however, that the high frequency output falls mildly as the impedance rises in a "Coag-Hard" mode if compared with a "Coag-Soft" mode. The difference in the output characteristic operates to differentiate the strength of the exfoliative component. In FIG. 8, characteristic curve c (thick solid line) shows the output characteristic when 50W is selected for the output power level in a "Coag-Hard" mode. The characteristic curve c clearly shows that the high frequency output of 50W is maintained up to the impedance level of about 2.5 kΩ but the high frequency output gradually falls beyond 2.5 kΩ and gets to about 25W, or a half of the former level, when the impedance rises to 5 kΩ.

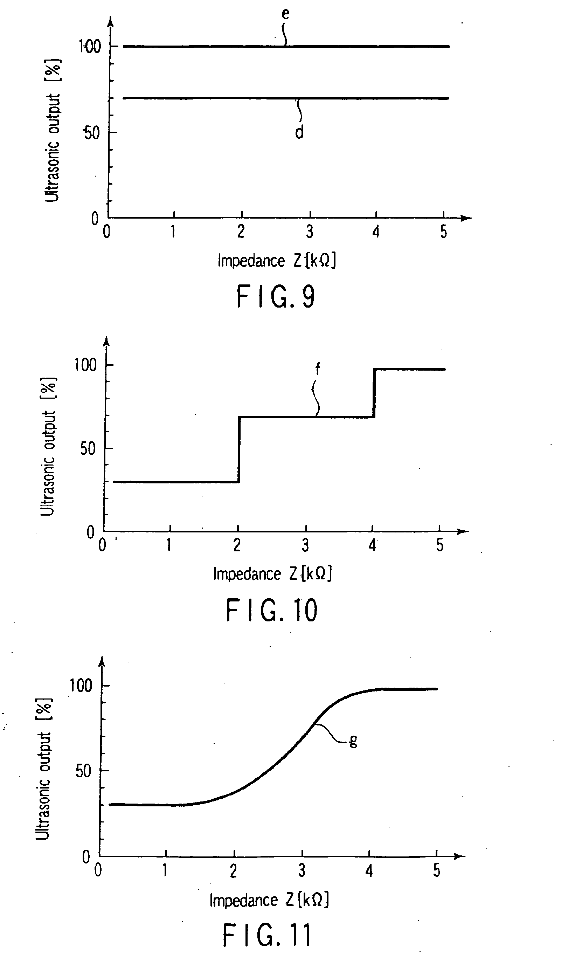

FIGS. 9 through 11 illustrate the output characteristic of the ultrasonic output as different examples. In FIGS. 9 through 11, the horizontal axis indicates the impedance at the time of high frequency output (the impedance between the hand piece 4 and the bipolar plate 9) and the vertical axis indicates the ultrasonic output. As for the magnitude (volume) of ultrasonic output, "100%" indicates the largest output level (the amplitude of ultrasonic oscillations is largest). The oscillation speed V of the treatment section 26 of the hand piece 4 is preferably within the range between about 7.4 m/s and 22.1 m/s when "100%" is selected. The oscillation speed V is defined by the formula shown below:

$$V = \pi \cdot x \cdot fr,$$

Where x represents the amplitude of ultrasonic oscillations (peak to peak value) and fr represents the resonance frequency of ultrasonic oscillations. Thus, if "100%" is selected, preferably, x is within the range between about 100 μm and 300 μm when fr is 23.5 kHz but it is within the range between 50 μm and 150 μm when fr is 47 kHz.

In FIG. 9, characteristic curve d shows the output characteristic when the output level of "70%" is selected in an "on" mode for the ultrasonic output. Characteristic curve e shows the output characteristic when the output level of "70%" is selected in a "max" mode for the ultrasonic output. A constant ultrasonic output level (amplitude of ultrasonic oscillations) is maintained regardless of the impedance value.

In FIG. 10, characteristic curve f shows the output characteristic in an "Auto" mode for the ultrasonic output as an example. As shown, the output characteristic is such that the ultrasonic output increases stepwise as the impedance rises. More specifically, the ultrasonic output is 30% up to the impedance level of 2 kΩ and 70% up to the impedance level between 2 kΩ and 4 kΩ whereas the ultrasonic output is held to 100% beyond the impedance level of 4 kΩ.

In FIG. 11, characteristic curve g shows the output characteristic in an "Auto" mode for the ultrasonic output as another example. As shown, the output characteristic is such that the ultrasonic output increases smoothly as the impedance rises. More specifically, the ultrasonic output is held to 30% up to the impedance level of about 1 kΩ and smoothly rises between about 1 kΩ and about 4 kΩ, whereas the ultrasonic output is held to 100% beyond the impedance level of about 4 kΩ.

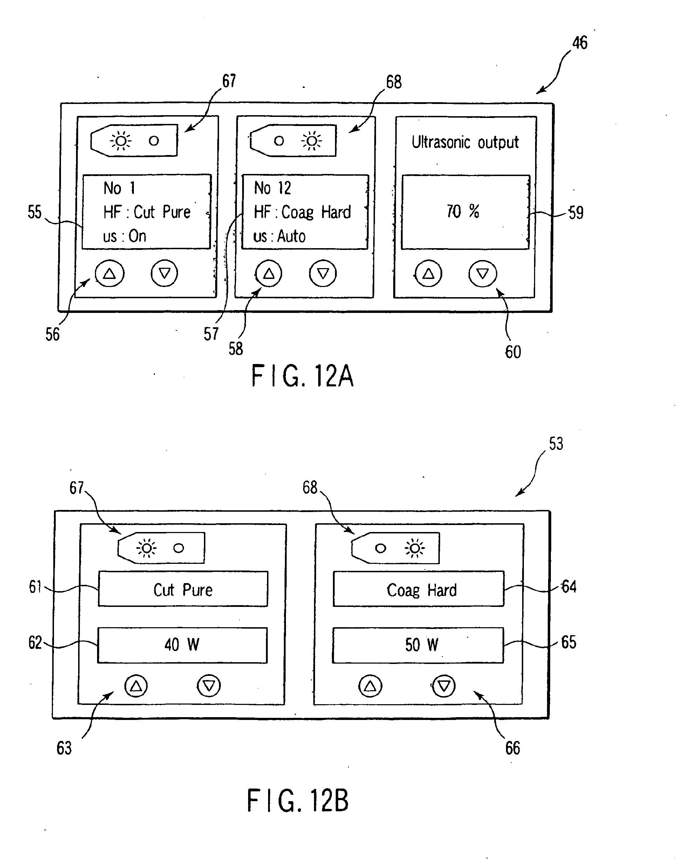

FIG. 12A is a schematic illustration of an image that can be displayed on the operation/display panel 46 of the ultrasonic drive unit 2. FIG. 12B is a schematic illustration of an image that can be displayed on the operation/display panel 53 of the high frequency drive unit 3.

The operation/display panel 46 displays a first output mode display section 55 and a first output mode selecting section 56.

The operation/display panel 46 also displays a second output mode display section 57 and a second output mode selecting section 58. Additionally, the operation/display panel 46 displays an ultrasonic output selection/display section 59 and an ultrasonic output selecting section 60. On the other hand, the operation/display panel 53 displays a first output mode display section 61, a first output selection/display section 62 and a first output selecting section 63. The operation/display panel 53 also displays a second output mode display section 64, a second output selection/display section 65 and a second output selecting section 66.

With the above-described display arrangement, the operator firstly selects the first output mode at the first output mode selecting section 56 of the operation/display panel 46 and then the second output mode at the second output mode selecting section 58 of the operation/display panel 46. As a result of these selections, the contents of the selected first output mode are displayed in the first output mode display section 55 while the contents of the selected second output mode are displayed in the second output mode display section 57.

FIG. 12A shows that "No. 1" listed in FIG. 6 is selected as the first output mode while "No. 12" also listed in FIG. 6 is selected as the second output mode. Then, the operator selects a value defining the (amplitude of the) ultrasonic output in an "On" mode for the ultrasonic output in the ultrasonic output selecting section 60 of the operation/display panel 46. In the instance of FIG. 12A, an ultrasonic output "70%" is selected (70% of the largest amplitude is selected as amplitude).

Then, a signal representing the selections made on the operation/display panel 46 is transmitted from the CPU 45 to the output control section 52 by way of the communication cable 5. Thus, the high frequency output mode of the first output mode (first high frequency output mode) selected in the first output mode selecting section 56 is displayed in the first output mode display section 61 of the operation/display panel 53 while the high frequency output mode of the second output mode (second high frequency output mode) selected in the second output mode selecting section 58 is displayed in the second output mode display section 64 of the operation/display panel 53.

Then, as shown in FIG. 12B, the selected output value ("40W" in this instance) of the first high frequency output mode selected in the first output selecting section 63 of the operation/display panel 53 is displayed in the first output selection/display section 62 while the selected output value ("50W" in this instance) of the second high frequency output mode selected in the second output selecting section 66 of the operation/display panel 53 is displayed in the second output selection/display section 65. Note that, in this embodiment, the first output mode corresponds to the first switch 32 of the hand piece 4 and the second output mode corresponds to the second output switch 33 of the hand piece 4.

Numbers 67, 68 representing the respective correspondences are displayed respectively near the first output mode display section 55 and the first output mode display section 61, and near the second output mode display section 57 and the second output mode display section 64.

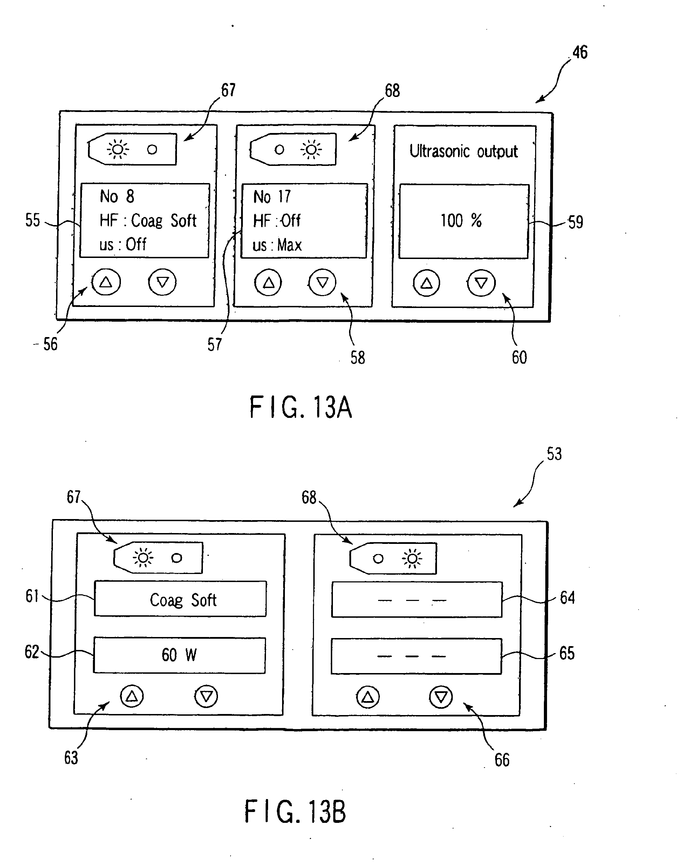

FIG. 13A is a schematic illustration of an image that can be displayed on the operation/display panel 46 for another setting of operation. FIG. 13B is a schematic illustration of another image that can be displayed on the other operation/display panel 53 for the setting of FIG. 13A. FIG. 13A shows that "No. 8" listed in FIG. 6 is selected as the first output mode while "No. 17" also listed in FIG. 6 is selected as the second output mode. It will be appreciated that the second output mode display section 64 and the second output selection/display section 65 of the operation/display panel 53 become automatically ineffective (and display "- - -") because the high frequency output is "Off" in the second output mode. On the other hand, the ultrasonic output selection/display section 59 of the operation/display panel 46 automatically displays "100%" because the ultrasonic output is "Off" in the first output mode and the ultrasonic output is "Max" in the second output mode.

Now, the treatment of living body tissue by means of the surgical instrument 1 of this embodiment will be described below.

Firstly, the bipolar plate 9 is brought into tight contact with the living body tissue that are the object of operation.

Then, the output conditions (the first output conditions, the second output conditions) of the surgical instrument 1 are defined by operating the operation/display panel 46 of the ultrasonic drive unit 2 and the operation/display panel 53 of the high frequency drive unit 3.

As the output conditions are defined, the first output condition and the second output condition are displayed respectively on the operation/display panel 46 and the operation/display panel 53. See FIGS. 12A, 12B, 13A, 13B. When the conditions are defined, the first output conditions are displayed in the first output mode display section 55, the first output mode display section 61 and the first output selection/display section 62. The second output conditions are displayed in the second output mode display section 57, the second output mode display section 64 and the second output selection/display section 65.

Then, the treatment section 26 of the hand piece 4 is brought into contact with the site of treatment of the living body tissue. As the first switch 32 of the hand switch 31 is turned on, the hand piece 4 is driven to operate under the first output conditions. Alternatively, as the second switch 33 of the hand switch 31 is turned on, the hand piece 4 is driven to operate under the second output conditions. Then, as a result, the living body tissue held in contact with the treatment section 26 of the hand piece 4 are treated by means of the ultrasonic output and/or the high frequency output.

Now, the effect of each of the output conditions will be described below.

Firstly, the output condition of the combination of a high frequency output mode "Off" and an ultrasonic output mode "On" or "Max" (No. 16 or No. 17 in FIG. 6) will be described. In this instance, only the ultrasonic output is realized. As the hand switch 31 is turned on, the ultrasonic transducer 12 of the hand piece 4 is driven by the ultrasonic transducer drive circuit 37.

As a result, the probe 25 starts producing ultrasonic oscillations and the living body tissue that are held in contact with the treatment section 26 of the probe 25 are hardened as blood coagulates due to the frictional heat generated by the ultrasonic oscillations and consequently incised. Note that the ultrasonic output level is held to the defined value (e.g., 70%) selected in the ultrasonic output selecting section 60 of the operation/display panel 46 in an "On" mode for the ultrasonic output whereas to the level of "100%" in a "Max" mode for the ultrasonic output. In other words, the ultrasonic output (the amplitude of ultrasonic oscillations) is held to a constant level when an "On" mode or a "Max" mode is selected for the ultrasonic output. Since a current control system is adopted as amplitude control system in this embodiment, the ultrasonic transducer 12 is driven with a constant current under control. In other words, the surgical instrument 1 operates for treatment like a conventional ultrasonic instrument for coagulation and incision.

Now, the output condition of the combination of each of the high frequency output modes and the ultrasonic output mode "Off" (No. 2, No. 5, No. 8, No. 11 and No. 14 in FIG. 6) will be described. In this instance, the ultrasonic output is not realized and hence only the high frequency output is produced.

As the hand switch 31 is turned on, an ON signal is transmitted from the CPU 45 of the ultrasonic transducer drive circuit 37 to the output control section 52 of the high frequency drive circuit 48. Then, the high frequency drive circuit 48 is driven to flow a high frequency current between the treatment section 26 of the hand piece 4 and the bipolar plate 9. As a result, blood in the living body tissue held in contact with the treatment section 26 is coagulated by the Joule's heat generated by the high frequency current and the living body tissue is incised. Thus, the surgical instrument 1 operates for treatment like a conventional monopolar type electric knife.

Now, the output condition of the combination of each of the high frequency output modes and the ultrasonic output mode "On" (No. 1, No. 4, No. 7, No. 10 and No. 13 in FIG. 6) will be described. In this instance, both the high frequency output and the ultrasonic output are produced simultaneously.

As the hand switch 31 is turned on, the ultrasonic transducer 12 of hand piece 4 is driven by the ultrasonic transducer drive circuit 37 to ultrasonically oscillate the probe 25. Additionally, an ON signal is transmitted from the hand switch 31 to the output control section 52 of the high frequency drive circuit 48 to flow a high frequency current between the treatment section 26 of the hand piece 4 and the bipolar plate 9.

As a result, the living body tissue that are brought into contact with the treatment section 26 are treated by both the ultrasonic output and the high frequency output. The ultrasonic transducer 12 is driven by a constant current under control and the ultrasonic output is maintained to the level (e.g., 70%) selected at the ultrasonic output selecting section 60 of the operation/display panel 46.

Note that the high frequency output shows the output characteristics as illustrated in FIGS. 7 and 8. More specifically, the selected high frequency output is maintained when electric impedance of the living body tissue is relatively low but lowered as the impedance rises.

Thus, when the electric impedance of the living body tissue is low (because, for example, a large volume of blood is adhering to the living body tissue), blood is coagulated and the living body tissue are incised satisfactorily by the high frequency output and, at the same time, the influence of cavitation (e.g., scattering of blood) from the front end of the probe due to the ultrasonic output can be minimized by selecting a low value (e.g., 30%) for the ultrasonic output.

When, on the other hand, the electric impedance of the body tissue is high (because, for example, the body tissue is in a very dry condition), it is possible to make blood coagulate and incise the living body tissue satisfactorily by selecting a high value (e.g., 100%) for the ultrasonic output if the high frequency output is lowered. Note that the treatment section 26 is prevented from adhering to and scorching the body tissue to realize a good coagulation of blood and a satisfactory incision as the treatment section 26 of the probe 25 ultrasonically oscillates regardless if the ultrasonic output is low or high.

Now, the output condition of the combination of each of the high frequency output modes and the ultrasonic output mode "auto" (No. 3, No. 6, No. 9, No. 12 and No. 15 in FIG. 6) will be described. In this instance, both the high frequency output and the ultrasonic output are produced simultaneously and the magnitude of the ultrasonic output changes as a function of the impedance that is observed when the high frequency output is produced.

As the hand switch 31 is turned on, the ultrasonic transducer 12 of hand piece 4 is driven by the ultrasonic transducer drive circuit 48 to ultrasonically activate the probe 25. Additionally, an ON signal is transmitted from the hand switch 31 to the output control section 52 of the high frequency drive circuit 48 to flow a high frequency current between the treatment section 26 of the hand piece 4 and the bipolar plate 9. As a result, the living body tissue that are brought into contact with the treatment section 26 are treated by both the ultrasonic output and the high frequency output.

At this time, a signal representing the magnitude of the impedance that is observed when the high frequency output is produced is transmitted from the output control section 25 of the high frequency drive circuit 48 to the CPU 45 of the ultrasonic transducer drive circuit 37. Then, the CPU 45 specifies the ultrasonic output condition as shown in FIG. 10 or FIG. 11 to the D/A converter 44, which D/A converter 44 generates a signal representing an electric current of a level that corresponds to the ultrasonic output condition specified by the CPU 45.

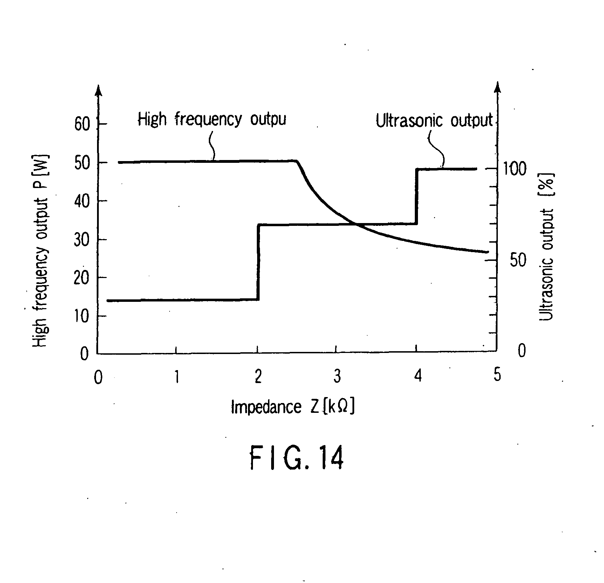

As a result, the magnitude of the ultrasonic output (amplitude of the ultrasonic oscillations) changes as a function of the magnitude of the impedance that is observed when the high frequency output is produced. Note that the output characteristic of the high frequency output is one indicated in FIGS. 7 and 8. More specifically, the selected high frequency output is maintained when the electric impedance of the living body tissue is relatively low but lowered as the impedance rises. If, for example, the output condition is that of No. 12 in FIG. 6 (a combination of the high frequency output "Coag-Hard" and the ultrasonic output "Auto"), the output characteristic will be the one illustrated in FIG. 14.

FIG. 14 is a graph illustrating electric impedance when 50W is selected for the high frequency output "Coag-Hard".

Referring to FIG. 14, when the electric impedance of the living body tissue is lower than about 2 kΩ (because, for example, a large volume of blood is adhering to the living body tissue), the high frequency output is 50W and the ultrasonic output is 30%. Then, blood is coagulated and the living body tissue are incised satisfactorily by the high frequency output and, at the same time, the influence of cavitation (e.g., scattering of blood) from the front end of the probe due to the ultrasonic output can be minimized.

When, on the other hand, the electric impedance of the living body tissue is about 4 kΩ or more (because, for example, the body tissue is in a very dry condition), the high frequency output is less than about 30W and the ultrasonic output is 100%. Then, it is possible to make blood coagulate and incise the living body tissue satisfactorily although the high frequency output is lowered.

When, finally, the electric impedance of the living body tissue is at an intermediate level between about 2 kΩ and 4 kΩ (because the living body tissue are in a normal condition), the high frequency output is about 30 to 50W and the ultrasonic output is 70%. Then, it is possible to make blood coagulate and incise the living body tissue satisfactorily by means of the high frequency output and the ultrasonic output. Note that the treatment section 26 is prevented from adhering to and scorching the body tissue to realize a good coagulation of blood and a satisfactory incision as the treatment section 26 of the probe 25 ultrasonically oscillates regardless of the level of the electric impedance.

The magnitude of the high frequency output and that of the ultrasonic output automatically change in response to the electric impedance of the living body tissue to be treated (and hence the type and the condition of the living body tissue to be treated). Therefore, it is not necessary to select the level of the high frequency output and that of the ultrasonic output according to the type and the condition of the living body tissue to be treated to consequently make the operation of the surgical instrument a simple one.

Note that first output condition and the second output condition of the surgical instrument 1 can be selected at will from the output conditions listed in FIG. 6. Therefore, the treatment can be performed depending on the type of operation and the propensity of the operator. Thus, the surgical instrument 1 can find a variety of applications.

The output condition of the combination of any of the output mode of the high frequency output and "Auto" of the ultrasonic output provides the following advantages.

The magnitude of the high frequency output and that of the ultrasonic output are automatically selected in response to the electric impedance of the living body tissue to be treated (and hence the type and the condition of the living body tissue to be treated). Therefore, it is no longer necessary to select the level of the high frequency output and that of the ultrasonic output depending on the type and the condition of the living body tissue to be treated by the operator to consequently make the operation of the surgical instrument a simple one.

Additionally, the output conditions of the surgical instrument 1 can be selected at will from the various possible output conditions (including one for producing only the high frequency output and one for producing only the ultrasonic output). Therefore, the treatment can be performed depending on the type of operation and the propensity of the operator. Thus, the surgical instrument 1 can find a variety of applications.

Now, the second embodiment of the present invention will be described below.

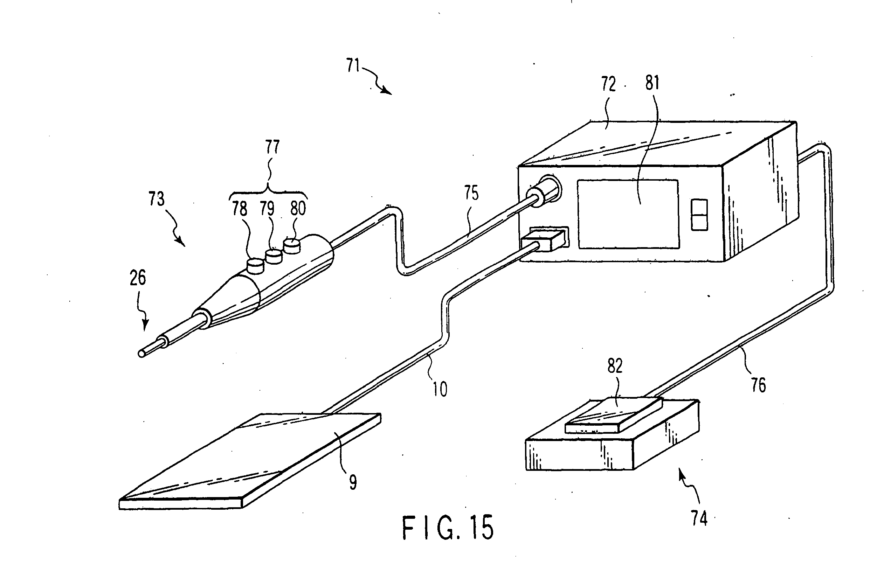

FIG. 15 is a schematic perspective view of the second embodiment of surgical instrument, showing the appearance thereof. Only the parts of the second embodiment that are different from the first embodiment will be described below.

The surgical instrument 71 of this embodiment comprises as principal components an ultrasonic/high frequency drive unit 72, a hand piece 73 and a foot switch 74. The hand piece 73 is connected to the ultrasonic/high frequency drive unit 72 by way of an output/SW connection cable 75. The ultrasonic/high frequency drive unit 72 is a unit realized by integrally combining the ultrasonic drive unit 2 and the high frequency drive unit 3 of the first embodiment. The foot switch 74 is connected to the ultrasonic/high frequency drive unit 72 by way of an SW connection cable 76. A bipolar plate 9 is connected to the ultrasonic/high frequency drive unit 72 by way of a connection cable 10. The hand piece 73 is provided with a first switch 78 and a second switch 79 that are collectively referred to as hand switch 77. While two hand switches are provided in this embodiment, the number of hand switches is by no means limited to two and more than two hand switches and may alternatively be provided depending on the functional features to be assigned to such switches.

The ultrasonic/high frequency drive unit 72 contains therein an ultrasonic transducer drive circuit 37 and a high frequency drive circuit 48 as shown in FIG. 5 and described above by referring to the first embodiment.

As in the case of the first embodiment, the CPU 45 of the ultrasonic transducer drive circuit 37 and the output control section 52 of the high frequency drive circuit 48 are connected to each other to transmit signals bidirectionally. The ultrasonic/high frequency drive unit 72 is provided with an operation/display panel 81 that is similar to any of the operation/display panels of the first embodiment.

The output/SW connection cable 75 is realized by combining the output connection cable 6, the SW connection cable 7 and the output connection cable 8 of the first embodiment. The foot switch 74 is equipped with a pedal switch 82. The SW connection cable 76 is connected to the CPU 45 of the ultrasonic transducer drive circuit 37.

The operation of the surgical instrument 71 having the above-described configuration for treating living body tissue will be described below.

Firstly, the bipolar plate 9 is brought into tight contact with the living body tissue.

Then, the output conditions of the surgical instrument 71 are defined by operating the operation/display panel 81 of the ultrasonic/high frequency drive unit 72. As the output conditions are defined, the output conditions are displayed on the operation/display panel 81.

Then, the treatment section 26 of the hand piece 73 is brought into contact with the site of treatment of the living body tissue. After that, the hand piece 73 is driven to operate as the first switch 78, the second switch 79 or the third switch 80 of the hand switch 77 is turned on. Alternatively, the hand piece 73 is driven to operate as the pedal switch 82 of the foot switch 74 is turned on.

Then, as a result, the living body tissue held in contact with treatment section 26 of the hand piece 73 are treated by means of the ultrasonic output and/or the high frequency output. The output conditions corresponding to the operation of one of the first, second and third switches 78, 79, 80 and that of the pedal switch 82 of the foot switch 74 can be selected at will on the operation/display panel 81 of the ultrasonic/high frequency drive unit 72. Any of the output conditions as listed in FIG. 6 can be selected in a manner as described above by referring to the first embodiment. The effects of each combinations of output conditions are the same as those described above by referring to the first embodiment.

Thus, the ultrasonic/high frequency drive unit 72 of this embodiment is realized by integrally combining the ultrasonic drive unit 2 and the high frequency drive unit 3 of the first embodiment and the output/SW connection cable 75 is realized by integrally combining the output connection cable 6, the SW connection cable 7 and the output connection cable 8 of the first embodiment.

Thus, the setting operation of the surgical instrument 71 is simple and can be performed in a short period of time. Additionally, the handleabilty of the hand piece 73 is improved for surgical treatments and either the hand switch 77 or the foot switch 74 can be selectively used at will. Thus, the surgical instrument 71 can find a variety of applications.

Now, the third embodiment of surgical instrument according to the invention will be described below.

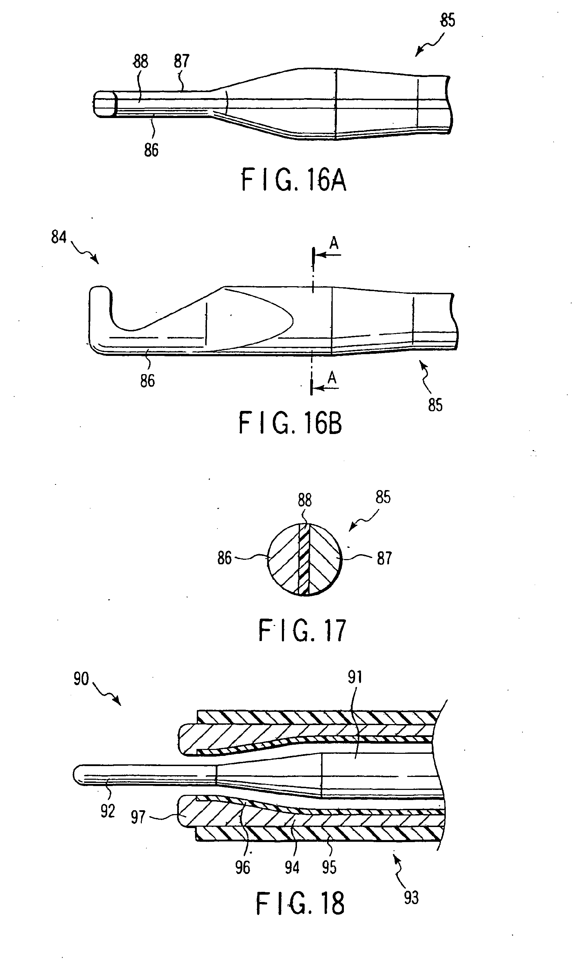

FIGS. 16A and 16B schematically illustrate the third embodiment of surgical instrument according to the present invention and comprising a hook type hand piece. FIG. 17 is a schematic cross sectional view of the treatment section 84 taken along line A-A in FIG. 16B. FIG. 18 is a schematic longitudinal cross sectional view of the treatment section of the third embodiment. Only the parts of the third embodiment that are different from the first embodiment will be described below.

The hand piece of this embodiment has a hook type transmission section 84 that operates as bipolar type electric knife. The probe 85 includes a first electrode member 86, a second electrode member 87 and an insulating member 88 to transmit ultrasonic oscillations. The first electrode member 86 and the second electrode member 87 are made of a metal material such as titanium, duralumin or stainless steel. The insulating member 88 is made of an electrically insulating ceramic material such as alumina or zirconia or an electrically insulating resin material such as PTFE or PEEK.

As shown in FIGS. 16A and 17, the first electrode member 86 and the second electrode member 87 are electrically insulated by the insulating member 88. An output connection cable 8 as described above by referring to the first embodiment is connected to the first electrode member 86, while a connection cable 10 as described above also by referring to the first embodiment is connected to the second electrode member 87. A bipolar plate 9 as described above by referring to the first embodiment is not required in this embodiment.

FIG. 18 is a schematic longitudinal cross sectional view of a needle type hand piece realized by modifying the third embodiment.

The probe 91 of this modified embodiment has an oblong and cylindrical tip section 92 and is adapted to transmit ultrasonic oscillations. The probe 91 is typically made of a metal material such as titanium, duralumin or stainless steel. Sheath 93 through which the probe 91 is made to extend includes a metal pipe 94, an insulating tube 95 covering the metal pipe 94 and an insulating member 96 buried in the inner periphery of the metal pipe 94. The metal pipe 94 is made of a metal material such as stainless steel, while the insulating tube 95 and the insulating member 96 are made of an electrically insulating resin material such as PTFE or PEEK.

A front end section 97 of the metal pipe 94 projects toward the front end side with respect to the insulating tube 95 and the insulating member 96.

In this embodiment, the tip section 92 of the probe 91 and the front end section 97 of the metal pipe 94 constitute the treatment section 90. An output connection cable 8 as described above by referring to the first embodiment is connected to the probe 91, while a connection cable 10 as described above also by referring to the first embodiment is connected to the metal pipe 94. A bipolar plate 9 as described above by referring to the first embodiment is not required in this embodiment.

The operation of the surgical instrument having the above-described configuration for treating living body tissue will be described below.

Firstly, the treatment section 84 (or the treatment section 90) of the hand piece is brought into tight contact with the living body tissue that are the object of operation. Then, as described above by referring to the first embodiment, the hand switch 31 is turned on to drive the hand piece. As a result, the probe 85 (or the probe 91) is driven to produce ultrasonic oscillations. In the case of the probe 85 of the hook type hand piece, a high frequency current flows between the first electrode member 86 and the second electrode member 87. Similarly, in the case of the probe 91 of the needle type hand piece, a high frequency current flows between the tip section 92 and the front end section 97 of the metal pipe 94.

As a result, the body tissue held in contact with the treatment section 84 (or the treatment section 90) is treated by the ultrasonic output and/or the high frequency output.

A combination of any of the output conditions of the high frequency output and any of the output conditions of the ultrasonic output as described above by referring to the first embodiment can also be used in this embodiment. Additionally, this embodiment provides advantages similar to those of the first embodiment that operates as monopolar type electric knife.

**Claims**

1. A surgical instrument which is adapted to perform a treatment of incision and coagulation on living body tissue by applying a high frequency current and ultrasonic vibration, comprising:

   a hand piece (4) equipped with a treatment section (26) arranged at the tip side thereof to treat the living body tissue and including a probe (25) to be supplied with the high frequency current and an ultrasonic transducer (12) rigidly secured to the probe (25) and adapted to activate the probe (25) by the ultrasonic vibration;
   a high frequency drive circuit (3) which is adapted to supply a high frequency current to the probe (25); and
   an ultrasonic transducer drive circuit (2) which is adapted to drive the ultrasonic transducer (12), **characterized by** comprising
   a control section (45, 52) which is adapted to control the amplitude of the ultrasonic vibration according to the magnitude of the electrical impedance value of the living body tissue as measured when applying the high frequency current and the ultrasonic vibration to the living body tissue while performing the treatment with the high frequency current.

2. The surgical instrument according to claim 1, **characterized in that**
   the control section (45, 52) is adapted to cause the high frequency drive circuit (3) to reduce the high frequency current as the impedance value increases, and is adapted to cause the ultrasonic transducer drive circuit (2) to increase the amplitude of the ultrasonic vibration applied to the treatment section (26) as the impedance value increases.

3. The surgical instrument according to claim 2, **characterized in that**
the vibration speed of the treatment section (26) is in a range between 7.4 m/s and 22.1 m/s when the amplitude of the ultrasonic vibration applied to the treatment section (26) is largest.

4. The surgical instrument according to claim 1, **characterized in that**
the control section (45, 52) is adapted to independently control the high frequency drive circuit (3) and the ultrasonic transducer drive circuit (2) for their respective outputs.

5. The surgical instrument according to claim 2, **characterized in that**
the control section (45, 52) is adapted to independently control the high frequency drive circuit (3) and the ultrasonic transducer drive circuit (2) for their respective outputs.

6. The surgical instrument according to claim 1, **characterized in that**
the hand piece (4) has at least a first switch (32) and a second switch (33); and
the first switch (32) is adapted to give a directive for driving the treatment section (26) in a first output mode, and
the second switch (33) is adapted to give a directive for driving the treatment section (26) in a second output mode;
the first switch (32) and the second switch (33) being adapted to be operated independently so as to supply a high frequency current and/or ultrasonic vibration to the treatment section (26) of the probe (25) for treating the living body tissue.

7. The surgical instrument according to claim 6, **characterized by** further comprising:

   a foot switch (74) for issuing a directive for driving the ultrasonic transducer drive circuit (2) and the high frequency drive circuit (3).

8. The surgical instrument according to claim 1, **characterized in that**
a mode in which the high frequency current is supplied to the probe (25) can be selected from a first high frequency output mode for a cut output containing virtually no hemostatic elements;
a second high frequency output mode for a cut output containing virtually some hemostatic elements;
a third high frequency output mode for coagulation output with weak separation elements;
a fourth high frequency output mode for coagulation output with strong separation elements;
a fifth high frequency output mode for coagulation output to be used for coagulating a wide area of tissue with a spray of sparks; and
a sixth high frequency output mode of not supplying any high frequency current to the probe (25), and
a mode in which the ultrasonic vibration is supplied to the probe (25) is selected from
a first ultrasonic output mode which maintains the selected value for the amplitude of the ultrasonic vibration;
a second ultrasonic output mode which changes the amplitude of the ultrasonic vibration being connected with the impedance value at the time of the high frequency output; and
a third ultrasonic output mode of not outputting the ultrasonic vibration to the probe (25),
the surgical instrument being adapted to be driven by the combination of the high frequency output mode and the ultrasonic output mode.

**Patentansprüche**

1. Chirurgisches Instrument, das dazu eingerichtet ist, eine Schnitt- und Koagulationsbehandlung an lebendem Körpergewebe durch Anlegen eines Hochfrequenzstroms und Ultraschallvibration auszuführen, aufweisend:

   ein Handteil (4), ausgestattet mit einem Behandlungsabschnitt (26), der an der Spitzenseite dieses angeordnet ist, um das lebende Körpergewebe zu behandeln, und enthaltend eine Sonde (25), die mit dem Hochfrequenzstrom versorgt wird, und einen Ultraschallwandler (12), der fest mit der Sonde (25) verbunden und dazu eingerichtet ist, die Sonde (25) durch die Ultraschallvibration zu aktivieren;
   einen Hochfrequenzsteuerungskreis (3), der dazu eingerichtet ist, der Sonde (25) einen Hochfrequenzstrom zuzuführen; und
   einen Ultraschallwandlersteuerungskreis (2), der dazu eingerichtet ist, den Ultraschallwandler (12) zu steuern, **dadurch gekennzeichnet, dass** es aufweist
   einen Steuerungsabschnitt (45, 52), der dazu eingerichtet ist, die Amplitude der Ultraschallvibration gemäß der Größe des elektrischen Impedanzwerts des lebenden Körpergewebes zu steuern, wie gemessen, wenn der Hochfrequenzstrom und die Ultraschallvibration an das lebende Körpergewebe angelegt werden während der Ausführung der Behandlung mit dem Hochfrequenzstrom.

2. Chirurgisches Instrument nach Anspruch 1, **dadurch gekennzeichnet, dass**
der Steuerungsabschnitt (45, 52) dazu eingerichtet ist, den Hochfrequenzsteuerungskreis (3) zu veranlassen, den Hochfrequenzstrom zu verringern, wenn sich der Impedanzwert erhöht, und dazu eingerichtet ist, den Ultraschallwandlersteuerungskreis (2) zu veranlassen, die Amplitude der an den Behandlungsabschnitt (26) angelegten Ultraschallvibration zu erhöhen, wenn sich der Impedanzwert erhöht.

3. Chirurgisches Instrument nach Anspruch 2, **dadurch gekennzeichnet, dass** die Vibrationsgeschwindigkeit des Behandlungsabschnitts (26) in einem Bereich zwischen 7,4 m/s und 22,1 m/s liegt, wenn die Amplitude der an den Behandlungsabschnitt (26) angelegten Ultraschallvibration am Größten ist.

4. Chirurgisches Instrument nach Anspruch 1, **dadurch gekennzeichnet, dass** der Steuerungsabschnitt (45, 52) dazu eingerichtet ist, unabhängig für ihre jeweiligen Ausgaben den Hochfrequenzsteuerungskreis (3) und den Ultraschallwandlersteuerungskreis (2) zu steuern.

5. Chirurgisches Instrument nach Anspruch 2, **dadurch gekennzeichnet, dass** der Steuerungsabschnitt (45, 52) dazu eingerichtet ist, unabhängig für ihre jeweiligen Ausgaben den Hochfrequenzsteuerungskreis (3) und den Ultraschallwandlersteuerungskreis (2) zu steuern.

6. Chirurgisches Instrument nach Anspruch 1, **dadurch gekennzeichnet, dass** das Handteil (4) mindestens einen ersten Schalter (32) und einen zweiten Schalter (33) aufweist; wobei der erste Schalter (32) dazu eingerichtet ist, eine Anweisung zum Steuern des Behandlungsabschnitts (26) in einem ersten Ausgabemodus auszugeben, und der zweite Schalter (33) dazu eingerichtet ist, eine Anweisung zum Steuern des Behandlungsabschnitts (26) in einem zweiten Ausgabemodus auszugeben; wobei der erste Schalter (32) und der zweite Schalter (33) dazu eingerichtet sind, unabhängig betrieben zu werden, um zum Behandeln des lebenden Körpergewebes, dem Behandlungsabschnitt (26) der Sonde (25) einen Hochfrequenzstrom und/oder Ultraschallvibration zuzuführen.

7. Chirurgisches Instrument nach Anspruch 6, **dadurch gekennzeichnet, dass** es ferner aufweist:

einen Fussschalter (74) zum Ausgeben einer Anweisung zum Steuern des Ultraschallwandlersteuerungskreises (2) und des Hochfrequenzsteuerungskreises (3).

8. Chirurgisches Instrument nach Anspruch 1, **dadurch gekennzeichnet, dass** ein Modus, in dem der Hochfrequenzstrom der Sonde (25) zugeführt wird, ausgewählt werden kann aus einem ersten Hochfrequenzausgabemodus für eine Schnittausgabe enthaltend nahezu keine blutstillenden Elemente;

einem zweiten Hochfrequenzausgabemodus für eine Schnittausgabe enthaltend einige blutstillende Elemente;

einem dritten Hochfrequenzausgabemodus für eine Koagulationsausgabe mit schwachen Teilungselementen;

einem vierten Hochfrequenzausgabemodus für eine Koagulationsausgabe mit starken Teilungselementen;

einem fünften Hochfrequenzausgabemodus für eine Koagulationsausgabe, die zum Koagulieren eines großen Gewebebereichs mit einer Funkensprühung verwendet wird; und

einem sechsten Hochfrequenzausgabemodus, bei dem der Sonde (25) kein Hochfrequenzstrom zugeführt wird, wobei

ein Modus, bei dem der Sonde (25) die Ultraschallvibration zugeführt wird, ausgewählt wird aus einem ersten Ultraschallausgabemodus, der den ausgewählten Wert für die Amplitude der Ultraschallvibration beibehält;

einem zweiten Ultraschallausgabemodus, der die Amplitude der Ultraschallvibration, die mit dem Impedanzwert zur Zeit der Hochfrequenzausgabe zusammenhängt, ändert; und

einem dritten Ultraschallausgabemodus, bei dem keine Ultraschallvibration an die Sonde (25) ausgegeben wird, wobei

das chirurgische Instrument dazu eingerichtet ist, durch die Kombination des Hochfrequenzausgabemodus und des Ultraschallausgabemodus gesteuert zu werden.

**Revendications**

1. Instrument chirurgical qui est adapté pour effectuer un traitement d'incision et de coagulation sur un tissu corporel vivant par application d'un courant haute fréquence et d'une vibration ultrasonore, comprenant :

une pièce à main (4) équipée d'une section de traitement (26) agencée sur le côté de pointe de celle-ci pour traiter le tissu corporel vivant et incluant une sonde (25) destinée à être alimentée avec le courant haute fréquence et un transducteur ultrasonore (12) fixé de façon rigide à la sonde (25) et adapté pour activer la sonde (25) par la vibration ultrasonore ;

un circuit d'entraînement haute fréquence (3) qui est adapté pour amener un courant haute fréquence jusqu'à la sonde (25) ; et

un circuit d'entraînement de transducteur ultrasonore (2) qui est adapté pour entraîner le transducteur ultrasonore (12), **caractérisé en ce que** comprenant

une section de commande (45, 52) qui est adaptée pour commander l'amplitude de la vibration ultrasonore en fonction de la grandeur de la valeur d'impédance électrique du tissu corporel vivant telle qu'elle est mesurée lors de l'application du courant haute fréquence et de la vibration ultrasonore au tissu corporel vivant, tout en effectuant le traitement avec le courant haute fréquence.

2.  Instrument chirurgical selon la revendication 1, **caractérisé en ce que**
    la section de commande (45, 52) est adaptée pour faire en sorte que le circuit d'entraînement haute fréquence (3) réduise le courant haute fréquence lorsque la valeur d'impédance augmente, et est adaptée pour faire en sorte que le circuit d'entraînement de transducteur ultrasonore (2) augmente l'amplitude de la vibration ultrasonore appliquée à la section de traitement (26) lorsque la valeur d'impédance augmente.

3.  Instrument chirurgical selon la revendication 2, **caractérisé en ce que**
    la vitesse de vibration de la section de traitement (26) est dans une plage entre 7,4 m/s et 22,1 m/s lorsque l'amplitude de la vibration ultrasonore appliquée à la section de traitement (26) est la plus grande.

4.  Instrument chirurgical selon la revendication 1, **caractérisé en ce que**
    la section de commande (45, 52) est adaptée pour commander indépendamment le circuit d'entraînement haute fréquence (3) et le circuit d'entraînement de transducteur ultrasonore (2) pour leurs sorties respectives.

5.  Instrument chirurgical selon la revendication 2, **caractérisé en ce que**
    la section de commande (45, 52) est adaptée pour commander indépendamment le circuit d'entraînement haute fréquence (3) et le circuit d'entraînement de transducteur ultrasonore (2) pour leurs sorties respectives.

6.  Instrument chirurgical selon la revendication 1, **caractérisé en ce que**
    la pièce à main (4) comporte un premier commutateur (32) et un deuxième commutateur (33) ; et
    le premier commutateur (32) est adapté pour donner une directive d'entraînement de la section de traitement (26) dans un premier mode de sortie, et
    le deuxième commutateur (33) est adapté pour donner une directive d'entraînement de la section de traitement (26) dans un deuxième mode de sortie ;
le premier commutateur (32) et le deuxième commutateur (33) étant adaptés pour être utilisés de manière indépendante de façon à délivrer un courant haute fréquence et/ou une vibration ultrasonore à la section de traitement (26) de la sonde (25) pour traiter le tissu corporel vivant.

7.  Instrument chirurgical selon la revendication 6, **caractérisé en ce que** comprenant en outre :

    un commutateur à pied (74) pour envoyer une directive d'entraînement au circuit d'entraînement de transducteur ultrasonore (2) et au circuit d'entraînement haute fréquence (3).

8.  Instrument chirurgical selon la revendication 1, **caractérisé en ce que**
    un mode dans lequel le courant haute fréquence est amené jusqu'à la sonde (25) peut être choisi parmi un premier mode de sortie haute fréquence pour une sortie d'incision ne contenant virtuellement pas d'éléments hémostatiques ;
    un deuxième mode de sortie haute fréquence pour une sortie d'incision contenant virtuellement quelques éléments hémostatiques ;
    un troisième mode de sortie haute fréquence pour une sortie de coagulation avec des éléments de séparation faibles ;
    un quatrième mode de sortie haute fréquence pour une sortie de coagulation avec des éléments de séparation forts ;
    un cinquième mode de sortie haute fréquence pour une sortie de coagulation destinée à être utilisée pour la coagulation d'une large superficie de tissu avec une pulvérisation d'étincelles ; et
    un sixième mode de sortie haute fréquence sans aucun apport de courant haute fréquence à la sonde (25), et
    un mode dans lequel la vibration ultrasonore est amenée jusqu'à la sonde (25) est choisi parmi un premier mode de sortie ultrasonore qui maintient la valeur sélectionnée pour l'amplitude de la vibration ultrasonore ;
    un deuxième mode de sortie ultrasonore qui modifie l'amplitude de la vibration ultrasonore en rapport avec la valeur d'impédance à l'instant de la sortie haute fréquence ; et
    un troisième mode de sortie ultrasonore sans délivrance en sortie de la vibration ultrasonore jusqu'à la sonde (25),
    l'instrument chirurgical étant adapté pour être entraîné par la combinaison du mode de sortie haute fréquence et du mode de sortie ultrasonore.

FIG.1

FIG.2

FIG.3

FIG.4

FIG. 5

16

List of Output Modes

| High frequency output | | | Ultrasonic output | No |
|---|---|---|---|---|
| Monopolar | Cut | Pure | On | 1 |
| | | | Off | 2 |
| | | | Auto | 3 |
| | | Blend | On | 4 |
| | | | Off | 5 |
| | | | Auto | 6 |
| | Coag | Soft | On | 7 |
| | | | Off | 8 |
| | | | Auto | 9 |
| | | Hard | On | 10 |
| | | | Off | 11 |
| | | | Auto | 12 |
| | | Spray | On | 13 |
| | | | Off | 14 |
| | | | Auto | 15 |
| Off | | | On | 16 |
| | | | Max | 17 |

FIG. 6

FIG. 7

FIG. 8

F I G. 9

F I G. 10

F I G. 11

46

67    68

| ☼ ○ | ○ ☼ | Ultrasonic output |
|---|---|---|
| No 1<br>HF : Cut Pure<br>us : On | No 12<br>HF : Coag Hard<br>us : Auto | 70 % |
| △  ▽ | △  ▽ | △  ▽ |

55

59

56    57    58    60

# FIG. 12A

53

67    68

| ☼ ○ | ○ ☼ |
|---|---|
| Cut Pure | Coag Hard |
| 40 W | 50 W |
| △  ▽ | △  ▽ |

61    64

62    65

63    66

# FIG. 12B

**FIG. 13A**

No 8
HF : Coag Soft
us : Off

No 17
HF : Off
us : Max

Ultrasonic output

100 %

**FIG. 13B**

Coag Soft

60 W

— — —

— — —

FIG. 14

F I G. 15

F I G. 16A

F I G. 16B

F I G. 17

F I G. 18

**EP 1 894 532 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002306507 A
- US 20030040672 A1
- WO 02056805 A2